# EUROPEAN PATENT APPLICATION

(11) **EP 2 870 965 A1**
(43) Date of publication of application: **13.05.2015**
(21) Application number: 14191790.6
(22) Date of filing: 05.11.2014
(51) Int. Cl.: A61K 31/428, A61K 9/08

(54) **Oral liquid pharmaceutical formulations of pramipexole**

(30) Priority: 06.11.2013 TR 201312846
(71) Applicant: Sanovel Ilac Sanayi ve Ticaret A.S., 34460 Istanbul (TR)
(72) Inventor: Türkyilmaz, Ali, 34460 Istanbul (TR); Güner, Dicle, 34460 Istanbul (TR); Yelken, Gülay, 34460 Istanbul (TR)
(74) Representative: Sevinç, Erkan

(57) **Abstract**

The present invention relates to an oral liquid pharmaceutical formulation comprising pramipexole or a pharmaceutically acceptable salt, solvate or hydrate thereof and sorbitol as co-solvent and one or more pharmaceutically acceptable excipients.

## Description

### Field of Invention

The present invention relates to an oral liquid pharmaceutical formulation comprising pramipexole or a pharmaceutically acceptable salt, solvate or hydrate thereof and sorbitol as co-solvent and one or more pharmaceutically acceptable excipients.

### Background of Invention

Pramipexole is a nonergot dopamine agonist. The chemical name of pramipexole is (S)-2-amino-4,5,6,7-tetrahydro-6-(propylamino)benzothiazole and its chemical structure is shown in the Formula 1.

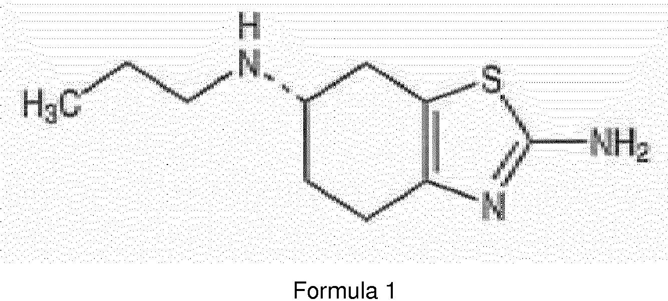

Pramipexole immediate release tablets are marketed under the name of Mirapex® and are indicated to be taken 3 times a day for oral administration and contain 0.125 mg, 0.25 mg, 0.5 mg, 1 mg, or 1.5 mg of pramipexole dihydrochloride monohydrate as active ingredient.

In prior art, pramipexole and its processes are described first in EP0186087B1 and it is known primarily for the treatment of schizophrenia and Parkinson's disease. In addition to tablet formulations, EP0186087B1 also discloses drops comprising pramipexole as an active pharmaceutical agent and parabens, anisole, ethanol, citric acid, sodium phosphate, sodium cyclamate and glycerol as excipients.

Solid oral dosage forms have the largest and most important role in the entire pharmaceutical formulations. However, there are a huge number of patients who have difficulties swallowing these dosage forms and oral liquid dosage forms are chosen by patients who are travelling or have little access to water or patients who are mentally retarded or nauseated. Unlike the solid dosage forms, oral liquid pharmaceutical forms require no dissolution so its absorption is rapid and unaffected by alterations in gastric pH. Thus, they deliver high concentration of active pharmaceutical agent to the intestinal epithelium and minimize first-pass metabolism.

On the other side, there are also a number of "challenges" surrounding the formulation and development of these forms such as stability, solubility and taste. While using a co-solvent, it may cause stability and bitter taste problems besides providing solubility. Moreover, active pharmaceutical agents in liquid forms are more susceptible to chemical and physical instability than the solid state. Trace amounts of impurities from active pharmaceutical agents or excipients and the pH of the solution may cause the degradation of active pharmaceutical agent and this may cause an increase in instability when the solution is consistently introduced into atmosphere.

In this invention, to fulfill requirements of an oral liquid pharmaceutical formulation of pramipexole, a formulation have been developed comprising pramipexole or a pharmaceutically acceptable salt, solvate or hydrate thereof and sorbitol as co-solvent and one or more pharmaceutically acceptable excipients. To further ensure the stability and suitable taste of formulation, butyl hydroxy anisole and butyl hydroxy toluene have been used as antioxidants.

### Description of Invention

The present invention relates to an oral liquid pharmaceutical formulation comprising pramipexole or a pharmaceutically acceptable salt, solvate or hydrate thereof and sorbitol as co-solvent and one or more pharmaceutically acceptable excipients.

In one embodiment, the oral liquid pharmaceutical formulation comprising pramipexole or a pharmaceutically acceptable salt, solvate or hydrate thereof may be an oral solution or a drop.

In this invention, the chemical stability of pramipexole has been provided throughout the shelf life by prevenmting precipitation and crystallization in the solution has been prevented while unpleasant taste problem has been overcome

In a preferred embodiment, pramipexole or a pharmaceutically acceptable salt, solvate or hydrate present in the oral liquid pharmaceutical formulation as pramipexole dihydrochloride monohydrate.

According to one embodiment, the amount of pramipexole dihydrochloride monohydrate is between 0.01 - 20.0%, preferably 0.01 - 10% and more preferably it is 0.01 - 5% by weight of total formulation.

In one embodiment, the oral liquid pharmaceutical formulation of this invention comprising one or more pharmaceutically acceptable excipient is selected from the group comprising co-solvents, solvents, antioxidants, microbial preservatives, buffering agents, aromatic agents, sweeteners and diluents.

In one embodiment, co-solvent used in the present invention is sorbitol.

The present invention provides an oral liquid pharmaceutical formulation comprising sorbitol as a co-solvent to achieve a desired solution for delivering the desired dose for a spoonful and a drop. Sorbitol is inert and compatible with other excipients. It is stable in air in the absence of catalysts and in any pH value. In this present invention, while sorbitol has ensured desired solubility and chemical stability, it has also prevented undesired colloidal precipitation and crystallization and ensured physical stability. A change in color or cloudiness of solutions may indicate chemical degradation; however, in this invention, the oral liquid pharmaceutical formulation prepared with sorbitol has not shown any of these indications. In addition, sorbitol also enhances the taste of formulation with its characteristic taste and contributes aromatic agents used in the formulation.

According to this embodiment, the amount of sorbitol used in oral liquid pharmaceutical formulation is in the range of 5 to 90 %, preferably 10 to 80 % and more preferably 10 to 60 % by weight of total formulation.

The stability assay has been performed by HPLC with gradient mobile phase at a wavelength of UV 264 nm at 40 ± 10 ºC of column temperature.

Precipitation analysis has been performed on the same ground which is a white flat ground lightened with a flash light parallel to the ground.

Color change analysis has been performed on the same ground which is a white flat ground lightened with a flash light parallel to the ground, and the color of the formulations is determined by using a color scale.

Suitable antioxidants may include but not limited to butylated hydroxyanisole (BHA), butylated hydroxytoluene (BHT), ascorbic acid, beta-carotene, alpha-tocopherol, propyl gallate, gentisic acid sodium ascorbate, sodium bisulfite, sodium metabisulfite, monothioglycero, cysteine, thioglycolate sodium, acetone sodium bisulfite, ascorbate (sodium/acid), bisulfite sodium, cystein/cysteinate HCl, dithionite sodium (Na hydrosulfite, Na sulfoxylate), gentisic acid, gentisic acid ethanolamine, glutamate monosodium, formaldehyde sulfoxylate sodium, metabisulfite potassium, metabisulfite sodium, monothioglycerol (thioglycerol), propyl gallate, sulfite sodium, tocopherol alpha, thioglycolate sodium or the mixtures thereof.

According to this embodiment, the amount of total antioxidants used in oral liquid pharmaceutical formulation is in the range of 0.01 to 4 % and preferably 0.01 to 2 % by weight of total formulation.

In this embodiment, the antioxidants used in the present invention are butyl hydroxy anisole and butyl hydroxy toluene. Preferably, butylated hydroxyanisole (BHA) and butylated hydroxytoluene (BHT) are used together.

Antioxidants are used in pharmaceutical solutions to enhance the stability of active agents and excipients that are susceptible to chemical degradation by oxidation. The oxidation of pramipexole dihydrochloride monohydrate in an oral liquid pharmaceutical formulation is difficult to control due to its chemical properties. In this present invention, to prevent oxidative degradation of pramipexole dihydrochloride monohydrate and further ensure the stability, BHA (butyl hydroxy anisole) and BHT (butyl hydroxy toluene) were used. While BHA inhibits free radical-induced drug decomposition and prevent spoilage by reacting with oxygen, BHT slows the development of off-flavours, odours and colour changes caused by oxidation. They have also both antiviral and antimicrobial activities against bacterial and viral contamination.

In this invention, with the synergistic effect of sorbitol, BHA and BHT, a stable oral liquid pharmaceutical formulation comprising pramipexole dihydrochloride monohydrate without any precipitation, degradation, unpleasant taste or microbial problems have been achieved.

Microbiologic control has been performed as described in European Pharmacopoeia 5.6.

According to this embodiment, the ratio of butyl hydroxy anisole (BHA) to butyl hydroxy toluene (BHT) is in the range of 0.001 to 200 (w/w), preferably 0.001 to 100 (w/w) and more preferably it is 0.001 to 20 (w/w).

Microbial preservatives may include but not limited to benzoic acid, boric acid, sorbic acid and their salts thereof, benzyl alcohol, benzalkonium chloride, parahydroxybenzoic acids and their alkyl esters, methyl and propyl parabens or their mixtures thereof.

Buffering agents may include but not limited to ascorbic acid, acetic acid, tartaric acid, citric acid monohydrate, trisodium citrate dehydrate, sodium citrate, potassium citrate, sodium phosphate, tricalcium phosphate, calcium carbonate, sodium bicarbonate, calcium phosphate, carbonated calcium phosphate, magnesium hydroxide, hydrochloric acid, sodium hydroxide or their mixtures thereof.

Co-solvents and solvents may include but not limited to glycerine, alcohols, propylene glycol, polyethylene glycol, water, ethanol, isopropyl alcohol or their mixtures thereof.

Aromatic agents may include but not limited to fruit aromas such as orange, banana, strawberry, cherry, wild cherry, lemon, etc., and other aromas such as cardamom, anis, mint, menthol, vanillin or their mixtures thereof.

Sweeteners may include but not limited to aspartame, sucralose, acesulfame-K, thaumatin, mogroside, saccharin and salts thereof, sodium cyclamate, glucose, sucrose, lactose, fructose, mannitol, sorbitol, lactitol, xylitol, erythritol, glycyrrhizin, monosodium glycyrrhizinate, monoamonium glycyrrhizinate, isomalt, glycerine, dextrose or their mixtures thereof.

Diluents may include but not limited to glucose syrup, glycerin, sorbitol and mannitol solutions, sucrose, sorbitol, xylitol, dextrose, fructose, maltitol, sugar potassium, aspartame, saccharine, saccharine sodium, spray dried or anhydrase lactose, mannitol, starch or their mixtures thereof.

According to an embodiment of the present invention, said oral liquid pharmaceutical formulation of pramipexole comprises;
a) 0.01 to 20 % by weight of pramipexole dihydrochloride monohydrate ,
b) 5 to 90 % by weight of sorbitol,
c) 0.01 to 2 % by weight of butyl hydroxy anisole (BHA)
d) 0.01 to 2 % by weight of butyl hydroxy toluene (BHT)
e) 0.001 to 15 % by weight of at least one microbial preservatives,
f) 0.01 to 20 % by weight of at least one buffering agent,
g) 5 to 90 % % by weight of at least one co-solvent or solvent,
h) 0.01 to 5 % by weight of at least one aromatic agent,
i) 0.001 to 4 % by weight of at least one sweetener.

### Example 1: oral drops

| **Ingredients** | **Amount %** |
|---|---|
| pramipexole dihydrochloride monohydrate | 0.01 - 10.0 |
| sorbitol | 10.00 - 80.00 |
| glycerine | 10.00 - 80.00 |
| sucralose | 0.10 - 2.00 |
| benzyl alcohol | 0.05 - 15.0 |
| butyl hydroxy anisole | 0.01 - 2.00 |
| butyl hydroxy toluene | 0.01 - 2.00 |
| aromatic agent | 0.10 - 1.00 |
| water / alcohol | 0.50 - 90.00 |
| Acid/ Base | q.s. |

| | |
|---|---|
| *q.s.: quantum sufficient* | |

The process of the formulations is carried out as follows: Pramipexole dihydrochloride monohydrate and all excipients are mixed respectively until it becomes a homogenous mixture. pH adjustment is performed in the mixture. The resulting mixture filtrated and rested. At the end of the rest, the mixture is filled into bottles under nitrogen gas by protecting from the light.

### Example 2: oral drops

| **Ingredients** | **Amount %** |
|---|---|
| pramipexole dihydrochloride monohydrate | 0.01 - 10.0 |
| sorbitol | 10.00 - 80.00 |
| glycerine | 10.00 - 80.00 |
| benzyl alcohol | 0.05 - 15.0 |
| trisodium citrate dihydrate | 0.01 - 10.0 |
| citric acid monohydrate | 0.01 - 10.0 |
| sucralose | 0.10 - 2.00 |
| butyl hydroxy anisole | 0.01 - 2.00 |
| butyl hydroxy toluene | 0.01 - 2.00 |
| aromatic agent | 0.10 - 1.00 |
| water / alcohol | 0.50 - 90.00 |

| | |
|---|---|
| *q.s.: quantum sufficient* | |

The process of the formulations is carried out as follows: Citrate buffer is prepared with trisodium citrate dihydrate and citric acid monohydrate in a tank containing water. All other excipients and pramipexole dihydrochloride monohydrate are added to this buffer solution and mixed until homogenous mixture is observed. The resulting mixture filtrated and rested. At the end of the rest, the mixture is filled into bottles under nitrogen gas by protecting from the light.

### Example 3: oral solution

| **Ingredients** | **Amount %** |
|---|---|
| pramipexole dihydrochloride monohydrate | 0.01 - 10.0 |
| sorbitol | 10.00 - 80.00 |
| glycerine | 10.00 - 80.00 |
| methyl paraben | 0.001 - 1.0 |
| propyl paraben | 0.001 - 1.0 |
| sucralose | 0.10 - 2.00 |
| butyl hydroxy anisole | 0.01 - 2.00 |
| butyl hydroxy toluene | 0.01 - 2.00 |
| aromatic agent | 0.10 - 1.00 |
| water / alcohol | 0.50 - 90.00 |
| Acid/ Base | q.s. |

| | |
|---|---|
| *q.s.: quantum sufficient* | |

The process of the formulations is carried out as follows: Sorbitol and glycerin added to tank containing water and mixed until homogenous mixture is observed. The mixture is heated. Methyl paraben and propyl paraben are added to this mixture and mixed until complete dissolution is achieved. The solution is cooled in room temperature. All other excipients and pramipexole dihydrochloride monohydrate are added to mixture in room temperature and mixed. pH adjustment is performed in the mixture. The resulting mixture filtrated and rested. At the end of the rest, the mixture is filled into bottles under nitrogen gas by protecting from the light.

### Example 4: oral solution

| **Ingredients** | **Amount %** |
|---|---|
| pramipexole dihydrochloride monohydrate | 0.01 - 10.0 |
| sorbitol | 10.00 - 80.00 |
| propylene glycol | 10.00 - 80.00 |
| methyl paraben | 0.001 - 1.0 |
| propyl paraben | 0.001 - 1.0 |
| monosodium glycyrrhizinate | 0.10 - 1.00 |
| butyl hydroxy anisole | 0.01 - 2.00 |
| butyl hydroxy toluene | 0.01 - 2.00 |
| aromatic agent | 0.10 - 1.00 |
| water / alcohol | 0.50 - 90.00 |
| Acid/ Base | q.s. |

| | |
|---|---|
| *q.s.: quantum sufficient* | |

The process of the formulations is carried out as follows: Sorbitol and propylene glycol added to tank containing water and mixed until homogenous mixture is observed. The mixture is heated. Methyl paraben and propyl paraben are added to this mixture and mixed until complete dissolution is achieved. The solution is cooled in room temperature. All other excipients and pramipexole dihydrochloride monohydrate are added to mixture in room temperature and mixed. pH adjustment is performed in the mixture. The resulting mixture filtrated and rested. At the end of the rest, the mixture is filled into bottles under nitrogen gas by protecting from the light.

### Example 5: oral solution

| **Ingredients** | **Amount %** |
|---|---|
| pramipexole dihydrochloride monohydrate | 0.01 - 10.0 |
| sorbitol | 10.00 - 80.00 |
| glycerine | 10.00 - 80.00 |
| methyl paraben | 0.001 - 1.0 |
| propyl paraben | 0.001 - 1.0 |
| trisodium citrate dihydrate | 0.10 - 10.00 |
| citric acid monohydrate | 0.10 - 10.00 |
| monoamonium glycyrrhizinate | 0.10 - 1.00 |
| butyl hydroxy anisole | 0.01 - 2.00 |
| butyl hydroxy toluene | 0.01 - 2.00 |
| sucralose | 0.10 - 2.00 |
| aromatic agent | 0.10 - 1.00 |
| water / alcohol | 0.50 - 90.00 |

| | |
|---|---|
| *q.s.: quantum sufficient* | |

The process of the formulations is carried out as follows: Citrate buffer is prepared with trisodium citrate dihydrate and citric acid monohydrate in a tank containing water. Sorbitol and glycerine is added to this mixture and mixed until homogenous mixture is observed. The mixture is heated. Methyl paraben and propyl paraben are added to this mixture and mixed until complete dissolution is achieved. The solution is cooled in room temperature. All other excipients and pramipexole dihydrochloride monohydrate are added to mixture in room temperature and mixed. The resulting mixture filtrated and rested. At the end of the rest, the mixture is filled into bottles under nitrogen gas by protecting from the light.

## Claims

1. An oral liquid pharmaceutical formulation comprising pramipexole or a pharmaceutically acceptable salt, solvate or hydrate thereof and sorbitol as co-solvent and one or more pharmaceutically acceptable excipients.

2. The oral liquid pharmaceutical formulation according to claim 1, wherein one or more pharmaceutically acceptable excipient is selected from the group comprising co-solvents, solvents, antioxidants, microbial preservatives, buffering agents, aromatic agents, sweeteners and diluents.

3. The oral liquid pharmaceutical formulation according to claim 1, wherein pramipexole or a pharmaceutically acceptable salt, solvate or hydrate is pramipexole dihydrochloride monohydrate.

4. The oral liquid pharmaceutical formulation according to claim 3, wherein pramipexole dihydrochloride monohydrate is in the range of 0.01 - 20.0%, preferably 0.01 - 10% more preferably 0.01 - 5% by weight of total formulation.

5. The oral liquid pharmaceutical formulation according to claim 1, wherein sorbitol is in the range of 5 to 90 %, preferably 10 to 80 % and more preferably 10 to 60 % by weight of total formulation.

6. The oral liquid pharmaceutical formulation according to claim 2, wherein the amount of total antioxidants is in the range of 0.01 to 4 % and preferably 0.01 to 2 % by weight of total formulation.

7. The oral liquid pharmaceutical formulation according to claim 6, wherein the antioxidants are butyl hydroxy anisole (BHA) and butyl hydroxy toluene (BHT).

8. The oral liquid pharmaceutical formulation according to claim 7, wherein the ratio of butyl hydroxy anisole (BHA) to butyl hydroxy toluene (BHT) is in the range of 1 to 200 (w/w), preferably 1 to 100 (w/w) and more preferably1 to 20 (w/w).

9. The oral liquid pharmaceutical formulation according to any preceding claim comprising;
a) 0.01 to 20 % by weight of pramipexole dihydrochloride monohydrate ,
b) 5 to 90 % by weight of sorbitol,
c) 0.01 to 2 % by weight of butyl hydroxy anisole (BHA)
d) 0.01 to 2 % by weight of butyl hydroxy toluene (BHT)
e) 0.001 to 15 % by weight of at least one microbial preservatives,
f) 0.01 to 20 % by weight of at least one buffering agent,
g) 5 to 90 % % by weight of at least one co-solvent or solvent,
h) 0.01 to 5 % by weight of at least one aromatic agent,
i) 0.001 to 4 % by weight of at least one sweetener.
